# EUROPEAN PATENT APPLICATION

(11) **EP 2 484 357 A1**
(43) Date of publication of application: **08.08.2012**
(21) Application number: 10820305.0
(22) Date of filing: 06.09.2010
(51) Int. Cl.: A61K 31/198, A21D 2/24, A23C 9/13, A23G 3/34, A23L 1/20, A23L 1/202, A23L 1/22, A23L 1/238, A23L 1/24, A23L 1/305, A23L 1/48, A23L 2/52, A61P 17/00, C12C 1/00

(54) **ORAL COMPOSITION FOR REDUCING SKIN ROUGHNESS**

(30) Priority: 30.09.2009 JP 2009226075
(71) Applicant: SHISEIDO COMPANY, LTD., Tokyo 104-8010 (JP)
(72) Inventor: NAKANO, Yusuke, Yokohama-shi Kanagawa 236-0004 (JP); ASHIDA, Yutaka, Yokohama-shi Kanagawa 236-0004 (JP); TOJO, Yosuke, Yokohama-shi Kanagawa 236-0004 (JP)
(74) Representative: Henkel, Breuer & Partner
(86) International application number: PCT/JP2010/065198
(87) International publication number: WO 2011/040185

(57) **Abstract**

Provided is a stable and safe oral composition that acts on the barrier function of the skin to reduce and/or prevent. An oral composition for reducing and an oral composition for preventing are provided, said compositions containing at least one compound selected from a group comprising D-glutamic acid and derivatives and/or salts thereof. Also provided are a pharmaceutical composition and/or a food composition containing the aforementioned oral composition for reducing or the aforementioned oral composition for preventing.

## Description

### TECHNICAL FIELD

The present invention relates to an oral composition for alleviating rough skin and an oral composition for preventing rough skin containing one or more compounds selected from the group consisting of D-glutamic acid and derivatives and/or salts thereof, and a method for alleviating rough skin and a method for preventing rough skin comprising a step of administering the compounds.

### BACKGROUND ART

Since a skin having rough skin exhibits an elevated transepidermal water loss (TEWL), a reduced barrier function of the skin has been considered to be one of the causes of the rough skin.

### PRIOR ART DOCUMENTS

### Non-Patent Document

Non-Patent document 1: "HYOJUN HIFU KAGAKU", 7th edition (IGAKUSHOIN)

### DISCLOSURE OF THE INVENTION

### PROBLEM TO BE SOLVED BY THE INVENTION

Conventional compositions for alleviating and/or preventing rough skin have been externally applied to the skin for aid in moisture retention in the skin rather than administered orally to inhibit a reduction in the barrier function of the skin.

Accordingly, it is necessary to develop a stable and safe oral composition capable of acting on the skin barrier function whereby alleviating and/or preventing the rough skin.

### MEANS FOR SOLVING THE PROBLEM

The present invention provides an oral composition for alleviating rough skin comprising one or more compounds selected from the group consisting of D-glutamic acid and derivatives and/or salts thereof.

The present invention provides an oral composition for preventing rough skin comprising one or more compounds selected from the group consisting of D-glutamic acid and derivatives and/or salts thereof.

The present invention provides a pharmaceutical composition comprising an oral composition for alleviating rough skin or an oral composition for preventing rough skin according to the invention.

The present invention provides a food composition comprising an oral composition for alleviating rough skin or an oral composition for preventing rough skin according to the invention.

The present invention provides a method for alleviating rough skin comprising a step of administering an oral composition for alleviating rough skin comprised of one or more compounds selected from the group consisting of D-glutamic acid and derivatives and/or salts thereof.

The present invention provides a method for preventing rough skin comprising a step of administering an oral composition for preventing rough skin comprised of one or more compounds selected from the group consisting of D-glutamic acid and derivatives and/or salts thereof.

In the method for alleviating rough skin and/or the method for preventing rough skin according to the invention, the oral composition for alleviating rough skin and/or oral composition for preventing rough skin according to the invention may be a pharmaceutical composition.

In the method for alleviating rough skin and/or the method for preventing rough skin according to the invention, the oral composition for alleviating rough skin and/or oral composition for preventing rough skin according to the invention may be a food composition.

As used herein, "rough skin" is one of the problems of the skin and means a condition where the texture of the skin defined by the skin surface dryness, sulcus cutis and crista cutis is disordered. The skin surface progressed the rough skin produces scales due to the disordered texture which results in an optical irregularity and this disorder can macroscopically be recognized.

As used herein, a "skin barrier function" is a function for keeping the skin in a normal condition which is a function for preventing a foreign body invasion or water loss through the skin.

As used herein, a "salt" of D-glutamic acid means any salt including a metal salt, an amine salt and the like, provided that the alleviating effect on rough skin of D-glutamic acid is not reduced. The metal salt may include an alkaline metal salt, an alkaline earth metal salt and the like. The amine salt may include triethylamine salt, benzylamine salt and the like.

As used herein, a "derivative" of D-glutamic acid means D-glutamic acid molecule covalently bound at its amino group, carboxyl group or side chain with any substituent group, provided that the rough skin alleviating effect of D-glutamic acid is not reduced. The substituent group includes, but is not limited to, a protective group, such as N-phenylacetyl group, 4,4'-dimethoxytrityl (DMT) group, etc.; a biological macromolecule, such as a protein, a peptide, a saccharide, and a lipid, a nucleic acid; a synthetic polymer, such as a polystyrene, a polyethylene, a polyvinyl, and a polyester; and a functional group such as an ester group. The ester group may include, for example, an aliphatic ester such methyl ester and ethyl ester, and an aromatic ester.

An amino acid has optical isomers which are L-form and D-form. A natural protein has L-amino acids bound by peptide bonds and only L-amino acids are employed excluding some exceptions such as a bacterial cell wall. Therefore, it has been considered that in a mammal including a human only L-amino acids are present and only L-amino acids are utilized (Kinouchi, T. et al., TANPAKUSHITSU KAKUSAN KOSO (PROTEIN, NUCLEIC ACID AND ENZYME), 50:453-460(2005), Lehninger Principles of Biochemistry [Vol.1] 2nd ed., pp132-147 (1993), Japanese-language translation, Hirokawa Shoten Ltd., Harper's Biochemistry, Original version, 22nd ed., pp21-30 (1991), Japanese-language translation Maruzen Co., Ltd.). Accordingly, only L-amino acids have mostly been employed as amino acids academically and industrially for a long time.

Exceptional cases where a D-amino acid is employed are, for example, a case of using as a raw material for an antibiotics produced by a microorganism, and, a case of a food additive employing a D-amino acid in a DL-amino acid mixture for the purpose of reducing cost of fractionating only an L-amino acid from a mixture of the L- and D-amino acids, which are obtained in equimolar amounts by synthesizing the amino acids. Nevertheless, there has been no case of using only D-amino acids industrially as bioactive substances.

D-Serine and D-aspartic acid have been studied to a comparatively advanced stage because of their higher ratios of D-forms. D-Serine is localized in a cerebrum and a hippocampus, and is known to be involved in an NMDA receptor regulating factor in the brain. D-Aspartic acid is localized in testis and pineal body, and reported to be involved in the regulation of hormone secretion (Japanese Patent Unexamined Publication No.2005-3558). However, the alleviating effect on rough skin of D-glutamic acid has not been elucidated.

The alleviating effect on rough skin of D-glutamic acid shown in the following Examples has not been known so far. Accordingly, an oral composition comprising D-glutamic acid according to the present invention is a novel invention.

Recently, it was reported that ddY mice were allowed to ingest freely a 10 mM aqueous solution of a D-amino acid for 2 weeks and then examined for the D-amino acid concentration in each organ, which was 3 to 1000 pmol per gland in a pineal body and 2 to 500 nmol per wet gram in a brain tissue (Morikawa, A. et al., Amino Acids, 32:13-20 (2007)). Based on this, the lower limit of daily intake of D-glutamic acid contained in a composition of the present invention described below was calculated.

As indicated in Examples described below, D-glutamic acid of the present invention exhibits an alleviating effect on rough skin in mice when given as an oral composition at concentrations of 1 to 10 mM aqueous solutions. Accordingly, the amount of D-glutamic acid contained in an oral composition for alleviating rough skin and a food composition of the present invention may be varied in a wide range, provided that D-glutamic acid is delivered to the skin tissue in vivo. When the composition of the present invention is an internal agent, the D-glutamic acid content may be 0.00001% by weight to 100% by weight. When the composition of the present invention is an internal agent, the D-glutamic acid content is preferably 0.00002% by weight to 80% by weight, most preferably 0.0002% by weight to 60% by weight. The lower limit of the daily dose of D-glutamic acid contained in the composition of the present invention may be 0.01 ng, preferably 0.1 ng, more preferably 1 ng per kg body weight.

The pharmaceutical composition of the present invention may further comprise, in addition to D-glutamic acid, salts of D-glutamic acid and/or derivatives capable of releasing D-glutamic acid by drug metabolizing enzymes and the like in vivo, one or more pharmaceutically acceptable additives, provided that the alleviating effect on rough skin of D-glutamic acid is not reduced. Such an additive includes, but is not limited to, a diluent and an extender, a binder and an adhesive, a lubricant, a glidant, a plasticizer, a disintegrant, a carrier solvent, a buffering agent, a colorant, a flavoring agent, a sweetener, a preservative and a stabilizer, an adsorbent, as well as other pharmaceutical additives known to those skilled in the art.

The food composition of the present invention may further comprise any other food-industrially acceptable components such as a seasoning, a colorant, and a preservative in addition to D-glutamic acid, salts of D-glutamic acid and/or derivatives capable of releasing D-glutamic acid by drug metabolizing enzymes and the like in vivo, provided that the alleviating effect on rough skin of D-glutamic acid is not reduced.

The food composition of the present invention may be any one employed conventionally as a food composition including, but not limited to, a candy, a cookie, bean paste, a French dressing, a mayonnaise, a French bread, a soy sauce, yogurt, dried seasoning powder for rice, seasoning sauce/sauce for natto (Japanese fermented soybean), natto, unrefined black vinegar.

### BRIEF DESCRIPTION OF THE DRAWINGS

Figure 1 is a graph showing the effect of an 11-day oral administration of D-glutamic acid on the skin barrier function in SDS-treated mice.
Figure 2 is a graph showing the effect of a 28-day oral administration of D-glutamic acid on the skin barrier function in SDS-treated mice.

### DESCRIPTION OF EMBODIMENTS

Examples of the present invention described below are intended only to exemplify the invention rather than to limit the technical scope thereof. The technical scope of the invention is limited only by the description in claims.

All references cited herein are incorporated by reference in its entirety.

The following experiments were conducted after obtaining an approval by Ethical Committee of Shiseido Research Center in compliance with a guideline by National Institute of Health (NIH) in the United States.

### Example 1

Alleviating effect on rough skin of short term administration of D-glutamic acid

### Methods

### Experimental animal

HR-1 hairless mice (males, seven-week old, Hoshino Laboratory Animals, Inc.) were subjected to the experiment. Each experimental group included seven to eight mice.

### Administration of amino acid

In order to examine the effect of the amino acid on the rough skin, 1 mM or 10 mM D-glutamic acid was given with drinking water. The negative control employed was drinking water containing no D-glutamic acid.

### Experiment on rough skin

An unwoven fabric soaked with a 10% SDS solution was brought into contact for five minutes with one side of the back of a mouse under anesthesia (hereinafter referred to as "SDS treatment") on the 10th and 11th days after starting the administration of D-glutamic acid. The SDS treatment may be conducted two to five times repetitively until the rough skin becomes evident. In this Example, the SDS treatments on the 10th and 11th days after starting the administration of D-glutamic acid are referred to as the first and the second SDS treatments, respectively. Also in the following transepidermal water loss measurements, an area treated with SDS is referred to as an "SDS treated area," while an area of the opposite side which was not treated with SDS is referred to as an "SDS non-treated area."

### Quantification of transepidermal water loss

In order to quantify the rough skin condition, the transepidermal water loss was measured immediately before the first SDS treatment (before SDS treatment) and two hours after the first and the second SDS treatments using a transepidermal water loss meter (Tewameter TM-300, manufactured by Courage + Khazaka electronic GmbH) in accordance with the instruction provided by the manufacturer. Here, the value obtained by subtracting the quantity of the transepidermal water loss of the SDS non-treated area from the quantity of the transepidermal water loss of the SDS treated area was regarded as a "Δ (delta) TEWL value."

### Results

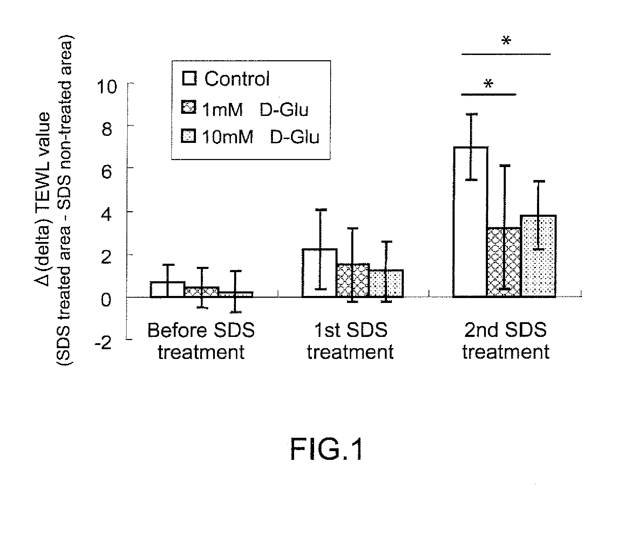

Figure 1 shows the results of the experiment examining the effect of the 11-day oral administration of D-glutamic acid on the SDS treatment in mice. The error bars for relevant experimental conditions are the standard deviations of the measured values of the results of the experiments seven to eight times under identical conditions. The asterisk (*) indicates p<5% by Dunnett's test.

The Δ (delta) TEWL values immediately before the first SDS treatment (before SDS treatment) were 0.7 g/m²/h in the negative control group, 0.4 g/m²/h in the 1 mM D-glutamic acid administration group, and 0.1 g/m²/h in the 10 mM D-glutamic acid administration group. The Δ (delta) TEWL values two hours after the first SDS treatment (the first treatment) were 2.2 g/m²/h in the negative control group, 1.5 g/m²/h in the 1 mM D-glutamic acid administration group, and 1.2 g/m²/h in the 10 mM D-glutamic acid administration group. The Δ (delta) TEWL values two hours after the second SDS treatment (the second SDS treatment) were 7.0 g/m²/h in the negative control group, 3.2 g/m²/h in the 1 mM D-glutamic acid administration group, and 3.8 g/m²/h in the 10 mM D-glutamic acid administration group. The Δ (delta) TEWL values 2 hours after the second SDS treatment exhibited significant difference between the 1 mM or 10 mM D-glutamic acid administration and the negative control. Based on these results, it was indicated that the repetitive SDS treatments cause the rough skin in mice but the rough skin can be prevented and/or alleviated by a prophylactic administration of D-glutamic acid.

### Example 2

Effect on alleviating rough skin of long term administration of D-glutamic acid

### Methods

The administration of the amino acid, the rough skin experiments and the quantification of the transepidermal water loss were conducted similarly to Example 1 using the HR-1 hairless mice described above. The amino acid employed was 10 mM L- or D-glutamic acid or L- or D-glutamine. The negative control employed was drinking water containing no amino acid. The SDS treatment was conducted on the 27th and 28th days after starting the administration of the amino acid. In this Example, the SDS treatments on the 27th and 28th days after starting the administration of the amino acid described above are referred to as the first and the second SDS treatments, respectively. The quantification of the transepidermal water loss was conducted two hours after the second SDS treatment.

### Results

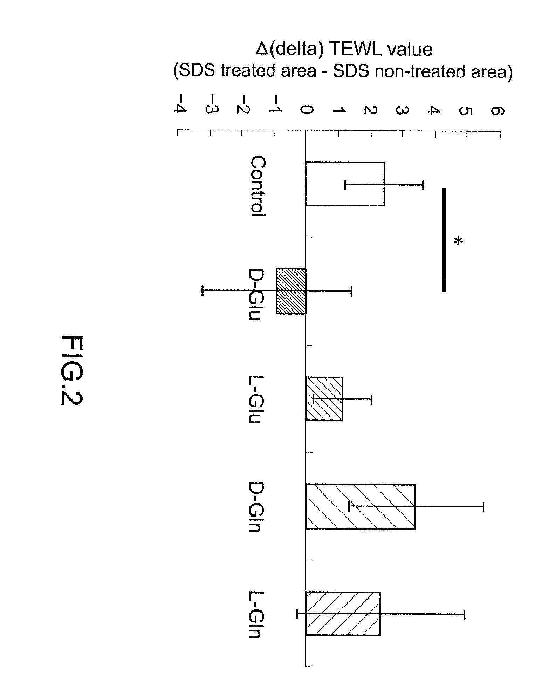

Figure 2 shows the results of the experiment examining the effect of the 28-day oral administration of D-glutamic acid on the SDS treatment in mice. The error bars for relevant experimental conditions are the standard deviations of the measured values of the results of the experiments repeated five times under identical conditions. The asterisk (*) indicates p<5% by Dunnett's test.

The Δ (delta) TEWL values two hours after the second SDS treatment (the second SDS treatment) were 2.4 g/m²/h in the negative control group, -0.9 g/m²/h in the D-glutamic acid administration group, 1. 1 g/m²/h in the L-glutamic acid administration group, 3.3 g/m²/h in the D-glutamine administration group, and 2.3 g/m²/h in the L-glutamine administration group. The Δ (delta) TEWL values two hours after the second SDS treatment exhibited a significant difference between the 10 mM D-glutamic acid administration and the negative control, while the L-glutamic acid, L-glutamine and D-glutamine administration exhibited no significant difference from the negative control. Based on these results, it was indicated that a long term administration of D-glutamic acid before the SDS treatment resul ts in a marked prevention and/or alleviation of the rough skin. Accordingly, it was suggested that a continuous ingestion of D-glutamic acid is effective in maintaining and/or promoting the skin barrier function.

Formulation examples of a tablet, a soft capsule, a granule, a beverage, a candy, a cookie, bean paste, a French dressing, a mayonnaise, a French bread, a soy sauce, yogurt, dried seasoning powder for rice, seasoning sauce/sauce for natto, natto, and unrefined black vinegar comprising D-glutamic acid based on the present invention are shown below. These formulation examples are all illustrative and not intended to restrict the technical scope of the present invention.

### Formulation Example 1 (Tablet)

| (Composition) | Content (mg/tablet) |
|---|---|
| D-Glutamic acid | 360.5 |
| Lactose | 102.4 |
| Calcium carboxymethyl cellulose | 29.9 |
| Hydroxypropyl cellulose | 6.8 |
| Magnesium stearate | 5.2 |
| Crystalline cellulose | 10.2 |
| | 515.0 |

### Formulation Example 2 (Tablet)

| (Composition) | Content (mg/tablet) |
|---|---|
| Sucrose ester | 70 |
| Crystalline cellulose | 74 |
| Methyl cellulose | 36 |
| Glycerin | 25 |
| Sodium D-glutamate | 475 |
| N-Acetylglucosamine | 200 |
| Hyaluronic acid | 150 |
| Vitamin E | 30 |
| Vitamin B6 | 20 |
| Vitamin B2 | 10 |
| α (alpha)-Lipoic acid | 20 |
| Coenzyme Q10 | 40 |
| Ceramide (Konnyaku extract) | 50 |
| L-Proline | 300 |
| | 1500 |

### Formulation Example 3 (Soft capsule)

| (Composition) | Content (mg/capsule) |
|---|---|
| Edible soybean oil | 530 |
| Eucommia ulmoides extract | 50 |
| Ginseng extract | 50 |
| Sodium D-glutamate | 100 |
| Royal jelly | 50 |
| Maca | 30 |
| GABA | 30 |
| Beeswax | 60 |
| Gelatin | 375 |
| Glycerin | 120 |
| Glycerin fatty acid ester | 105 |
| | 1500 |

### Formulation Example 4 (Soft capsule)

| (Composition) | Content (mg/capsule) |
|---|---|
| Brown rice germ oil | 659 |
| Sodium D-glutamate | 500 |
| Resveratrol | 1 |
| Lotus germ extract | 100 |
| Elastin | 180 |
| DNA | 30 |
| Folic acid | 30 |
| | 1500 |

### Formulation Example 5 (Granule)

| (Composition) | Content (mg/pack) |
|---|---|
| Sodium D-glutamate | 400 |
| Vitamin C | 100 |
| Soybean isoflavon | 250 |
| Reduced lactose | 300 |
| Soybean oligosaccharide | 36 |
| Erythritol | 36 |
| Dextrin | 30 |
| Flavoring agent | 24 |
| Citric acid | 24 |
| | 1200 |

### Formulation Example 6 (Beverage)

| (Composition) | Content (g/60 mL) |
|---|---|
| Eucommia ulmoides extract | 1.6 |
| Ginseng extract | 1.6 |
| D-glutamic acid | 0.3 |
| Reduced maltose syrup | 28 |
| Erythritol | 8 |
| Citric acid | 2 |
| Flavoring agent | 1.3 |
| N-Acetylglucosamine | 1 |
| Sodium hyaluronate | 0.5 |
| Vitamin E | 0.3 |
| Vitamin B6 | 0.2 |
| Vitamin B2 | 0.1 |
| α (alpha)-Lipoic acid | 0.2 |
| Coenzyme Q10 | 1.2 |
| Ceramide (Konnyaku extract) | 1.4 |
| L-proline | 2 |
| Purified water | Remainder |
| | 60 |

### Formulation Example 7 (Candy)

| (Composition) | Content (% by weight) |
|---|---|
| Sugar | 50 |
| Syrup | 48 |
| D-Glutamic acid | 1 |
| Flavoring agent | 1 |
| | 100 |

### Formulation Example 8 (Cookie)

| (Composition) | Content (% by weight) |
|---|---|
| Weak flour | 45.0 |
| Butter | 17.5 |
| Granulated sugar | 20.0 |
| Sodium D-glutamate | 4.0 |
| Egg | 12.5 |
| Flavoring agent | 1.0 |
| | 100.0 |

### Method for producing Formulation Example 8 (cookie)

Granular sugar was added in portions to butter while stirring, to which an egg, sodium D-glutamate and a flavor were added and stirred. After mixing thoroughly, uniformly sleved weak flour was added and stirred at a low speed, and allowed to stand as a bulk in a refrigerator. Thereafter, it was molded and baked for 15 minutes at 170°C (degrees Celsius) to obtain a cookie.

### Formulation Example 9 (Bean paste)

| (Composition) | Content (g) |
|---|---|
| Soybean | 1000 |
| Malted rice | 1000 |
| Salt | 420 |
| D-Glutamic acid | 158 |
| Water | Remainder |
| | 4000 |

### Method for producing Formulation Example 9 (Bean paste)

Malted rice is mixed thoroughly with a salt. Washed soybeans are soaked overnight in three times its volume of water, which are then drained off, and new water is added while boiling, and poured into a colander to collect the broth (tanemizu fluid), to which D-glutamic acid is dissolved at 10% w/v. The boiled beans are minced immediately, combined with malted rice mixed with salt, to which the tanemizu fluid containing D-glutamic acid dissolved therein is added and kneaded evenly to obtain a clay-like hardness. Dumplings are made and stuffed in a container compactly without forming any voids, and the surface of the content is smoothened and sealed with a plastic film. After 3 months, the content is transferred to a new container and the surface is smoothened and sealed with a plastic film. Instead of adding D-glutamic acid to the tanemizu fluid, a malted rice producing a large amount of D-glutamic acid may be employed. Such malted rice can be selected by quantifying D-glutamic acid by the method described in Japanese Patent Unexamined Publication No. 2008-185558. Alternatively, a commercially available bean paste can be supplemented with D-glutamic acid or a salt thereof.

### Formulation Example 10 (French dressing)

| (Composition) | Content (g) |
|---|---|
| Salad oil | 27.7 |
| Vinegar | 30.0 |
| Sodium chloride | 0.9 |
| D-Glutamic acid | 0.4 |
| Pepper | 1.0 |
| | 60.0 |

### Method for producing Formulation Example 10 (French dressing)

Vinegar is combined with sodium chloride and D-glutamic acid, and then stirred thoroughly to be dissolved. Salad oil is added to the mixture and the mixture is stirred thoroughly and then pepper is added.

### Formulation Example 11 (Mayonnaise)

| (Composition) | Content (g) |
|---|---|
| Salad oil | 134.5 |
| Vinegar | 5 |
| Sodium chloride | 0.9 |
| D-glutamic acid | 0.5 |
| Egg yolk | 18 |
| Sugar | 0.2 |
| Pepper | 0.9 |
| | 160.0 |

### Method for producing Formulation Example 11 (Mayonnaise)

An egg yolk (room temperature) is combined with vinegar, sodium chloride, D-glutamic acid and pepper, and stirred thoroughly using a whipping apparatus. Stirring is continued while adding salad oil in portions to form an emulsion. Finally, sugar is added and the mixture is stirred.

### Formulation Example 12 (French bread)

| (Composition) | Content (g) |
|---|---|
| Extra-strength flour | 140 |
| Weak flour | 60 |
| Sodium chloride | 3 |
| Sugar | 6 |
| Sodium D-glutamate | 2 |
| Dry yeast | 4 |
| Warm water | 128 |
| | 343 |

### Method for producing Formulation Example 12 (French bread)

Warm water is combined with 1 g of sugar and dry yeast, which is then allowed to undergo a pre-fermentation. Extra-strength flour, weak flour, sodium chloride, 5 g of sugar and sodium D-glutamate are placed in a bowl, into which the pre-fermented yeast is placed. After kneading thoroughly into a ball-like dough, a primary fermentation is conducted at 30°C (degrees Celsius). The dough is kneaded again and allowed to stand, and then shaped into suitably forms, which are subjected to a final fermentation using an electronic fermentation machine. After forming coupes, baking is conducted for 30 minutes in an oven at 220°C (degrees Celsius).

### Formulation Example 13 (Soy sauce)

| (Composition) | Content (g) |
|---|---|
| Commercially available soy sauce | 997 |
| Sodium D-glutamate | 3 |
| | 1000 |

### Method for producing Formulation Example 13 (Soy sauce)

Commercially available soy sauce is supplemented with sodium D-glutamate, and stirred thoroughly. Instead of adding D-glutamic acid or a salt thereof, malted rice producing a large amount of D-glutamic acid may be employed for fermenting soy sauce. Such malted rice can be selected by quantifying D-glutamic acid by the method described in Japanese Patent Unexamined Publication No. 2008-185558.

### Formulation Example 14 (Yogurt)

| (Composition) | Content (g) |
|---|---|
| Milk | 897 |
| L. bulgaricus | 50 |
| S. thermophilus | 50 |
| Sodium D-glutamate | 3 |
| | 1000 |

### Method for producing Formulation Example 14 (Yogurt)

Fermentation is conducted at 40 to 45°C (degrees Celsius). Other commercially available fermentation seed organisms may be employed and commercially available yogurt may be supplemented with sodium D-glutamate. Instead of adding D-glutamic acid or a salt thereof, a seed organism producing a large amount of D-glutamic acid may be employed. Such an organism can be selected by quantifying D-glutamic acid by the method described in Japanese Patent Unexamined Publication No. 2008-185558.

### Formulation Example 15 (Dried seasoning powder for rice)

| (Composition) | Content (g) |
|---|---|
| Sodium D-glutamate | 50 |
| Laver | 15 |
| Sodium L-glutamate | 10 |
| Sodium chloride | 2 |
| Roasted sesame | 10 |
| Dried mackerel shavings | 10 |
| Sugar | 1 |
| Soy sauce | 2 |
| | 100 |

### Formulation Example 16 (Seasoning, Sauce for natto)

| (Composition) | Content (g) |
|---|---|
| Commercially available sauce for natto | 9.96 |
| Sodium D-glutamate | 0.04 |
| | 10 |

### Formulation Example 17 (Natto)

| (Composition) | Content (g) |
|---|---|
| Commercially available natto | 19.9 |
| Sodium D-glutamate | 0.1 |
| | 20 |

### Method for producing Formulation Example 17 (Natto)

Instead of adding D-glutamic acid or a salt thereof, an organism producing a large amount of D-glutamic acid may be employed for producing natto. Such an organism can be selected by quantifying D-glutamic acid by the method described in Japanese Patent Unexamined Publication No. 2008-185558.

### Formulation Example 18 (Unrefined black vinegar)

| (Composition) | Content (g) |
|---|---|
| Commercially available unrefined black vinegar | 998 |
| D-glutamic acid | 2 |
| | 1000 |

### Method for producing Formulation Example 18 (Unrefined black vinegar)

Instead of adding D-glutamic acid or a salt thereof, an organism producing a large amount of D-glutamic acid may be employed for producing vinegar, black vinegar or unrefined vinegar. Such an organism can be selected by quantifying D-glutamic acid by the method described in Japanese Patent Unexamined Publication No. 2008-185558.

## Claims

1. An oral composition for alleviating rough skin comprising one or more compounds selected from the group consisting of D-glutamic acid and derivatives and/or salts thereof.

2. An oral composition for preventing rough skin comprising one or more compounds selected from the group consisting of D-glutamic acid and derivatives and/or salts thereof.

3. A pharmaceutical composition comprising an oral composition according to claim 1 or 2.

4. A food composition comprising an oral composition according to claim 1 or 2.
